# EUROPEAN PATENT APPLICATION

(11) **EP 4 678 171 A1**
(43) Date of publication of application: **14.01.2026**
(21) Application number: 24187623.4
(22) Date of filing: 10.07.2024
(51) Int. Cl.: A61K 31/18, A61K 31/421, A61P 27/02

(54) **SYSTEMICALLY-ADMINISTERED CXCL8 INHIBITORS FOR USE IN THE PREVENTION OR TREATMENT OF OCULAR MUCOUS MEMBRANE PEMPHIGOID AND/OR ORAL MUCOUS MEMBRANE PEMPHIGOID**

(71) Applicant: Dompé farmaceutici SpA, 20122 Milano (IT)
(72) Inventor: ARAMINI, Andrea, 67100 L'Aquila (IT); BRANDOLINI, Laura, 67100 L'Aquila (IT); GIORGIO, Cristina, 67100 L'Aquila (IT); DETTA, Nicola, 80131 Napoli (IT); BIANCHINI, Gianluca, 67100 L'Aquila (IT); PATZELT, Sabrina, 23538 Lübeck (DE); LUDWIG, Ralf, 23538 Lübeck (DE); SCHMIDT, Enno, 23538 Lübeck (DE)
(74) Representative: Dompé farmaceutici Spa

(57) **Abstract**

The invention relates to systemically-administered CXCL8 inhibitors useful in the prevention and/or treatment of ocular mucous membrane pemphigoid and/or oral mucous membrane pemphigoid.

## Description

### TECHNICAL FIELD

The invention relates to CXCL8 inhibitors for use in the prevention and/or treatment of ocular mucous membrane pemphigoid (OcMMP) and/or oral mucous membrane pemphigoid, wherein said CXCL8 inhibitors are administered systemically.

### BACKGROUND OF THE INVENTION

Mucous membrane pemphigoid (MMP) is a systemic cicatrizing autoimmune disease that primarily affects orificial mucous membranes, such as the conjunctiva, the nasal cavity, the oropharynx and the genitalia.

About 75% of MMP patients generate antibodies against BP180 (type XVII collagen) and 25% of MMP patients against laminin 332. In less than 5% of MMP patients, antibodies against type VII collagen or α6β4 integrin are detected (Domloge-Hultsch, N., et al., J Clin Invest, 1992. 90(4): p. 1628-33; Oyama, N., et al., Br J Dermatol, 2006. 154(1): p. 90-8; Schmidt, E., et al., Br J Dermatol, 2001. 145(5): p. 778-83).

Ocular involvement occurs in about 70% of all MMP cases and ocular MMP is the leading cause of cicatrizing conjunctivitis in developed countries.

Linear immunoglobulin A disease, mucosal dominated epidermolysis bullosa acquisita, and anti-laminin 332/anti-epiligrin/anti-laminin 5 pemphigoid are encompassed by ocular MMP (OcMMP).

The progressive inflammatory and scarring nature of ocular MMP leads to severe visual impairment in 30% of affected eyes and bilateral blindness in 20%.

Ocular MMP is often associated with oral mucosa lesions including desquamative gingivitis, vesicles, erosions covered by pseudomembranes and ulcers.

In some cases of MMP, only the oral mucosa is involved.

The underlying pathophysiological mechanism of the disease is a type-2 hypersensitivity reaction against the basal epithelial membrane of the conjunctiva.

In particular, conjunctival involvement is critical since the autoantibody-induced inflammation results in conjunctival scarring that may even progress after the inflammatory process has halted and leads to visual impairment and blindness.

Early diagnosis and appropriate treatment are of paramount importance to avoid inflammatory and infectious complications, as well as possible visual loss.

Ocular MMP management is aimed at controlling the immune-mediated inflammatory disease preventing fibrosis and progression of the disease. Georgoudis, P. et al, "Ocular Mucous Membrane Pemphigoid: Current State of Pathophysiology, Diagnostics and Treatment", Ophthalmol. Ther. (2019) 8, 5-17, discloses that a stepladder approach is used to select immunosuppressive agents and to escalate treatment, depending on disease severity (mild, moderate, severe). The medications used are dapsone, sulphapyridine, sulphasalazine, azathioprine (AZA), methotrexate (MTX), mycophenolate mofetil (MMF), cyclophosphamide, and short courses of oral steroids.

CD20 monoclonal antibodies, TNFα, inhibitors, and intravenous immunoglobulin (IVIg) are used to treat disease in patients non-responsive to conventional immunosuppressants. Treatment recommendations are also discussed in Schmidt et al., J Eur Acad Dermatol Venereol 2021, 35: 1926-48. The therapeutic mainstay are high-dose systemic corticosteroids supplemented with potentially corticosteroid sparing agents such as azathioprine, mycophenoles, dapsone, antibiotics with anti-inflammatory activity such as doxycycline, high-dose-intravenous immunoglobulins, and the anti-CD20 antibody rituximab.

The current treatment armamentarium is frequently not effective and associated with severe adverse events.

Thus, there is still a high need for effective and safe therapies.

Several possible mechanisms have been proposed underlying the development of antibody-mediated diseases MMP.

CXCL8 (interleukin-8, IL-8) is an endogenous chemotactic factor produced by most nucleated cells such as fibroblasts, macrophages, endothelial and epithelial cells.

As reported, the biological activity of CXCL8 is mediated by the interaction of CXCL8 with CXCR1 and CXCR2 membrane receptors belonging to the family of seven transmembrane receptors and expressed on the surface of human neutrophils and of several types of T-cells (L. Xu et al., J. Leukocyte Biol., 57, 335, 1995). Although CXCR1 activation is known to play a crucial role in CXCL8-mediated chemotaxis, it has been supposed that also CXCR2 activation could play a pathophysiological role in chronic inflammatory diseases. Different CXCL8 inhibitors have been developed and are well known to the skilled person.

WO2000/024710 discloses N-(2-aryl-propionyl)-sulfonamides, having inhibitory activity of neutrophils chemotaxis and degranulation induced by interleukin-8, and their use in the prevention and treatment of tissue damage due to the exacerbate recruitment of polymorphonuclear neutrophils (leukocytes PMN) at the inflammatory sites, in particular in the treatment of psoriasis, rheumatoid arthritis, ulcerative colitis, acute respiratory insufficiency, idiopathic fibrosis, glomerulonephritis.

WO2005/090295 discloses (R)-2-[4-(trifluoromethanesulfonyloxy)phenyl]propionic acid derivatives, which are used as inhibitors of the chemotaxis of polymorphonucleate and mononucleate cells, particularly in the treatment of neutrophils-dependent pathologies. The use of said compounds in the treatment of psoriasis, ulcerative colitis, melanoma, angiogenesis, chronic obstructive pulmonary disease (COPD), bullous pemphigo, rheumatoid arthritis, idiopathic fibrosis, glomerulonephritis and in the prevention and treatment of damages caused by ischemia and reperfusion is also disclosed.

WO2010/031835 discloses 2-aryl-propionic acids and derivatives, substituted in the 4 position by 2-amino-heterocycles as good CXCL8-induced chemotaxis inhibitors, useful in the prevention and treatment of tissue damage due to the exacerbated recruitment of polymorphonucleated neutrophils (PMN leukocytes) at inflammation sites. The use of said compounds in the treatment of transient cerebral ischemia, damages caused by ischemia and reperfusion, bullous pemphigo, rheumatoid arthritis, idiopathic fibrosis and glomerulonephritis is also disclosed.

The present invention is aimed at providing an effective treatment of MMP, in particular ocular and oral MMP.

### SUMMARY OF THE INVENTION

The invention is directed to CXCL8 inhibitors for use in the prevention and/or treatment of ocular mucous membrane pemphigoid (OcMMP) and/or oral mucous membrane pemphigoid in a subject, wherein said CXCL8 inhibitors are administered systemically.

The invention is also directed to pharmaceutical compositions comprising a CXCL8 inhibitor and at least one pharmaceutically acceptable excipient or carrier for use in the prevention and/or treatment of ocular mucous membrane pemphigoid and/or oral mucous membrane pemphigoid in a subject, wherein said pharmaceutical compositions are administered systemically.

The invention is also directed to a method of preventing and/or treating ocular mucous membrane pemphigoid and/or oral mucous membrane pemphigoid in a subject, which comprises administering an effective amount of one or more CXCL8 inhibitors to a subject in need thereof, wherein said one or more CXCL8 inhibitors are administered systemically.

The invention is also directed to the use of a CXCL8 inhibitor in the manufacture of a medicament for the prevention and/or treatment of ocular mucous membrane pemphigoid and/or oral mucous membrane pemphigoid in a subject, wherein said CXCL8 inhibitor is administered systemically.

### BRIEF DESCRIPTION OF FIGURES

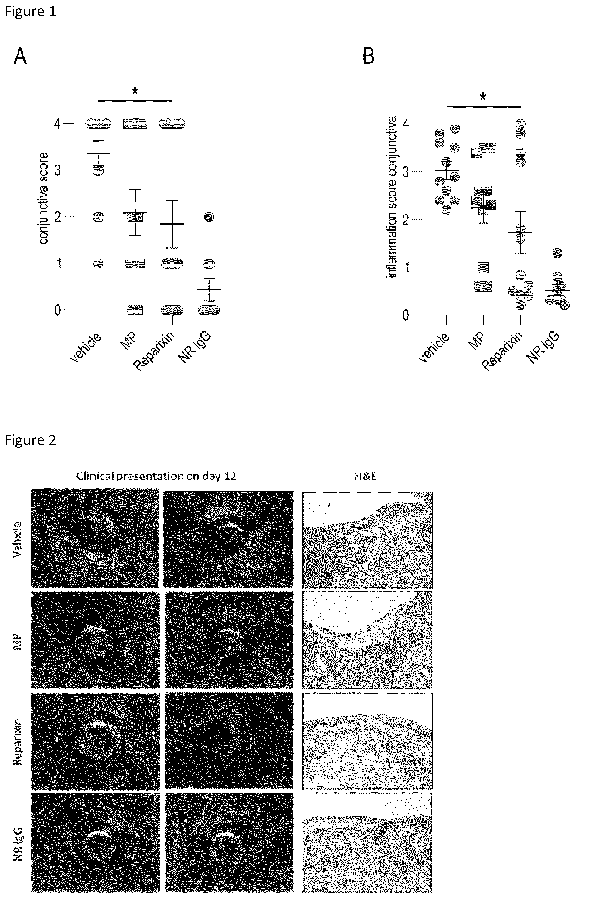

**Figure 1** shows in panel A) the effect of reparixin and MP systemic treatment on palpebral conjunctival split formation on day 12, expressed as conjunctiva score. Data are presented as means +/- standard deviation. For day 12 there is a statistically significant difference between the vehicle group and reparixin (p=0.0326). Data are based on 11-14 mice per group, except for NR IgG (n=10). The asterisk indicates statistical significance (ANOVA with Holm Sidaks method for multiple comparisons); and in panel B) the semi-quantification of subepithelial inflammatory infiltrate based on H&E stained biopsies of the palpebral conjunctiva. Data are presented as means +/- standard deviation. For day 12 there is a statistically significant difference between the vehicle group and reparixin (p=0.0110). Data are based on 11-13 mice per group, except for NR IgG (n=9). The asterisk indicates statistical significance (ANOVA with Holm Sidaks method for multiple comparisons).
**Figure 2** shows representative clinical presentations of the eyes and H&E stained sections of the palpebral conjunctiva obtained 12 days after the initial anti-mLAMα3 IgG injection.

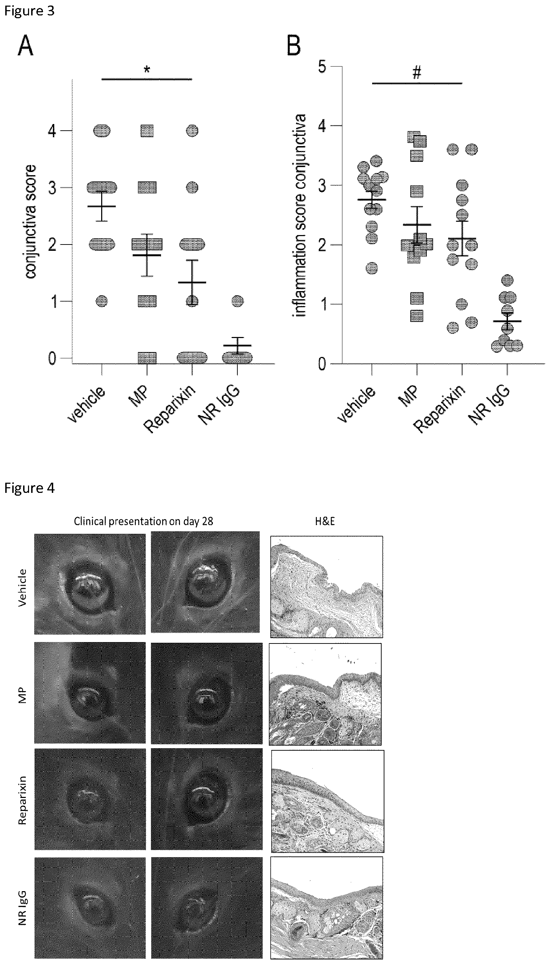

**Figure 3** shows in panel A) the effect of reparixin and MP systemic treatment on palpebral conjunctival split formation on day 28, expressed as conjunctiva score. Data are presented as means +/- standard deviation. For day 28 there is a statistically significant difference between the vehicle group and reparixin (p=0.0289). Data are based on 11-13 mice per group, except for NR IgG (n=9). The asterisk indicates statistical significance (ANOVA with Holm Sidaks method for multiple comparisons); and in panel B) the semi-quantification of subepithelial inflammatory infiltrate based on H&E stained biopsies of the palpebral conjunctiva. Data are presented as means +/- standard deviation. At day 28 reparixin significantly decreased the inflammatory infiltrate when only compared to vehicle treated mice (p=0.0323). Data are based on 11-13 mice per group, except for NR IgG (n=9). The rhombus indicates statistical significance by Unpaired t test with Welch's correction (no correction for multiple comparisons).
**Figure 4** shows the representative clinical presentations of the eyes and H&E stained sections of the palpebral conjunctiva obtained 28 days after the initial anti-mLAMα3 IgG injection.

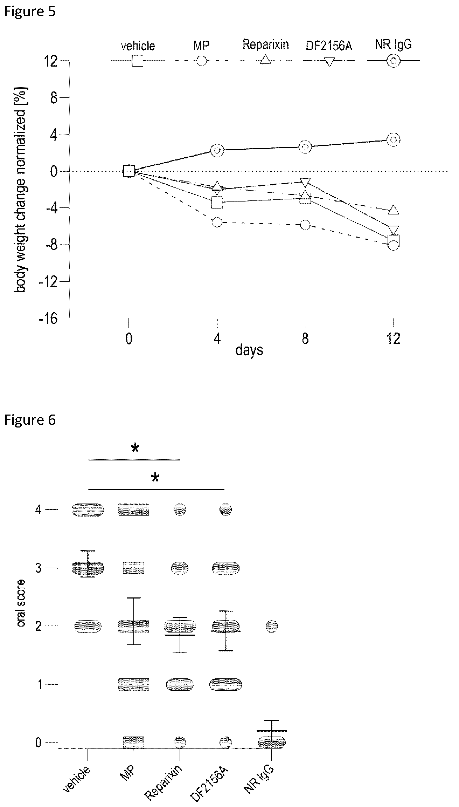

**Figure 5** shows the effect of DF2156A, reparixin and MP treatment on the body weight over 12 days, expressed as percentage of weight change in comparison to the weight on day 0. Data are presented as means +/- standard deviation. Data are based on 13-14 mice per group, except for NR IgG (n=10). No statistically significant differences were seen between body weight in treatment group compared to vehicle treated mice. Mixed-effects analysis with Dunnett's method for multiple comparisons.
**Figure 6** shows the effect of DF2156A, reparixin and MP treatment on severity of oral lesions on day 12, expressed as oral score. Data are presented as means +/- standard deviation. For day 12 there is a statistically significant difference between the vehicle group and reparixin (p=0.0329) and DF2156A (p=0.0428). Data are based on 13-14 mice per group, except for NR IgG (n=10). The asterisk indicates statistical significance (ANOVA with Holm Sidaks method for multiple comparisons).

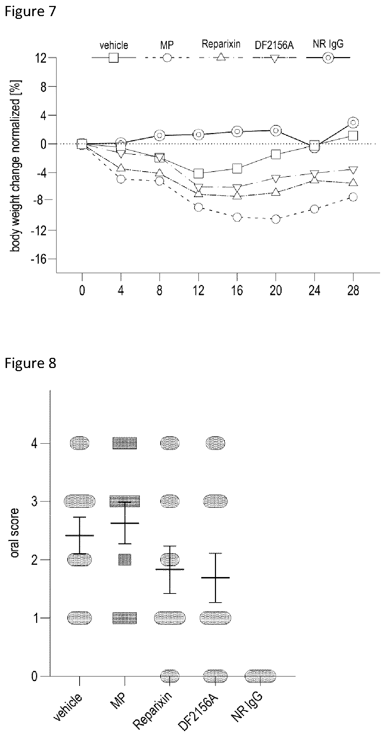

**Figure 7** shows the effect of DF2156A, reparixin and MP treatment on the body weight over 28 days, expressed as percentage of weight change in comparison to the weight on day 0. Data are presented as means +/- standard deviation. Data are based on 11-14 mice per group, except for NR IgG (n=9). MP treatment reduced the body weight (compared to day 0) of the mice compared to vehicle treated mice on day 4 (p=0.0164), day 16 (p=0.0053), day 20 (p=0.0017), and day 24 (p=0.0296). Mixed-effects analysis with Dunnett's method for multiple comparisons.
**Figure 8** shows the effect of DF2156A, reparixin and MP treatment on severity of oral lesions on day 28, expressed as oral score. Data are presented as means +/- standard deviation. Data are based on 11-13 mice per group, except for NR IgG (n=9). ANOVA with uncorrected Fishers LSD method (no correction for multiple comparisons).

### DETAILED DESCRIPTION OF THE INVENTION

It has been surprisingly found that the systemic administration of CXCL8 inhibitors, preferably the oral administration, is effective in the prevention and/or treatment of ocular mucous membrane pemphigoid (OcMMP) and oral mucous membrane pemphigoid.

Accordingly, the invention is directed to a CXCL8 inhibitor for use in the prevention and/or treatment of ocular mucous membrane pemphigoid (OcMMP) and/or oral mucous membrane pemphigoid in a subject, wherein said CXCL8 inhibitor is administered systemically to the subject.

According to a preferred embodiment, said CXCL8 inhibitor is administered orally to the subject.

According to a preferred embodiment, the invention is directed to a CXCL8 inhibitor for use in the prevention and/or treatment of ocular mucous membrane pemphigoid in a subject, wherein said CXCL8 inhibitor is administered systemically to the subject.

According to a preferred embodiment, said CXCL8 inhibitor is administered orally to the subject.

According to a further preferred embodiment, the invention is directed to a CXCL8 inhibitor for use in the prevention and/or treatment of oral mucous membrane pemphigoid in a subject, wherein said CXCL8 inhibitor is administered systemically to the subject.

According to a preferred embodiment, said CXCL8 inhibitor is administered orally to the subject.

According to a further preferred embodiment, the invention is directed to a CXCL8 inhibitor for use in the prevention and/or treatment of ocular mucous membrane pemphigoid and oral mucous membrane pemphigoid in a subject, wherein said CXCL8 inhibitor is administered systemically to the subject. According to a preferred embodiment, said CXCL8 inhibitor is administered orally to the subject.

The terms "treatment" and "prevention" as used herein refer to the eradication/amelioration or prevention/delay in onset, respectively, of the disorder being treated or of one or more of the symptoms associated thereof, notwithstanding the fact that the patient may still be afflicted with the underlying disorder.

The term "CXCL8 inhibitor" in accordance with the present invention means any compound able to inhibit the biological activity of CXCL8.

Methods for determining the inhibition of the biological activity of CXCL8 and for classifying a compound as "CXCL8 inhibitor" are known in the art and are described, for example, in Moriconi et al., J. Med. Chem. 2007, 50, 3984-4002, and in Brandolini et al., Scientific Reports (2019) 9: 11729.

Preferably, a CXCL8 inhibitor according to the present invention is a CXCL8 receptor(s) inhibitor.

Preferably said CXCL8 receptor(s) inhibitor is a CXCR1- or a CXCR1/2-inhibitor, which inhibits the activity of CXCL8 mediated by CXCR1 receptor or mediated by both the CXCR1 and CXCR2 receptors.

Said CXCL8 receptor(s) inhibitor preferably inhibits the binding of CXCL8 to the CXCR1 receptor (CXCR1 receptor inhibitor) or to both the CXCR1 and CXCR2 receptors (dual CXCR1 and CXCR2 receptors inhibitor), or prevents or blocks the intracellular signaling activated by the binding of CXCL8 to the CXCR1 receptor (CXCR1 receptor inhibitor) or to both the CXCR1 and CXCR2 receptors (dual CXCR1 and CXCR2 receptors inhibitor).

According to a preferred embodiment, the CXCL8 receptor(s) inhibitor is an antagonist of the CXCR1 receptor or an antagonist of both CXCR1 and CXCR2 receptors.

According to another preferred embodiment, the CXCL8 receptor(s) inhibitor is an allosteric inhibitor or an orthosteric antagonist of CXCR1 receptor or of both CXCR1 and CXCR2 receptors.

Alternatively, the CXCL8 receptor(s) inhibitor preferably binds to CXCL8, thus preventing its binding to its receptors.

Said CXCL8 receptor(s) inhibitor is preferably able to inhibit in an in-vitro assay at least 60%, preferably at least 70%, more preferably at least 80%, even more preferably at least 90% of PMNs chemotaxis induced by 1 nM CXCL8 at a concentration equal or below 500 nM, preferably below 100 nM.

More preferably, the CXCL8 receptor(s) inhibitor according to the invention has an IC₅₀ value towards CXCR1 receptor in the low nanomolar range, preferably lower than 10 nanomolar, more preferably in the range 0.02-5 nanomolar.

According to a further preferred embodiment, said CXCL8 inhibitor is selected from small molecules, peptides and antibodies, more preferably it is a small molecule.

The term "small molecule" refers to an organic compound having a molecular weight of 900 Daltons or lower.

CXCL8 inhibitors, in particular CXCL8 receptor(s) inhibitors, as defined above, are well known in the art.

To date, several CXCL8 inhibitors, such as small molecules, peptides and antibodies, have been disclosed, many of which are currently undergoing clinical trials or are used in therapy (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78, Sitaru et al., Internal and Emergency Medicine (2023) 18: 1647-1664). Preferably, the CXCL8 inhibitor according to the invention is selected from the group comprising (or consisting of):
- the anti-CXCL-8 antibodies ABCream, BMS-986253 and ABX-IL-8;
- RP-72, PAC-G-31-P, SCH-N,
- Navarixin, having formula: 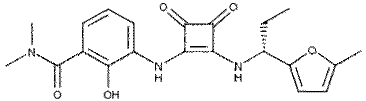
- SX-517, having formula: 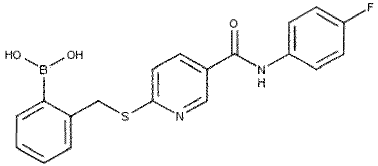
- SX-576, having formula: 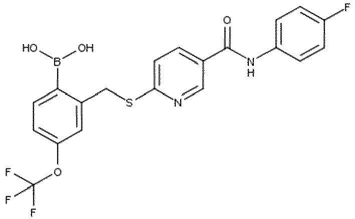
- SX-682, having formula: 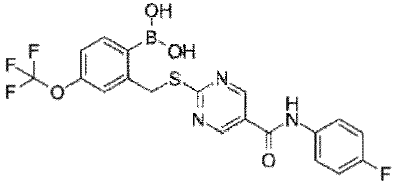
- compounds having formula: 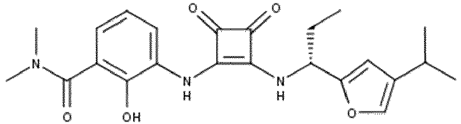 or 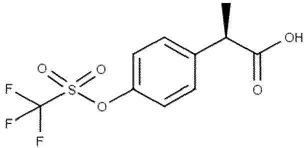 or 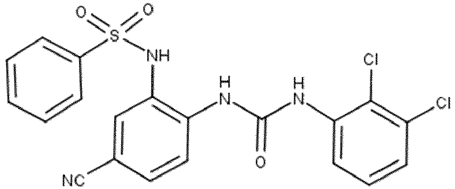 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) described hereinbelow.

According to a preferred embodiment, said CXCL8 inhibitor has general formula (I): 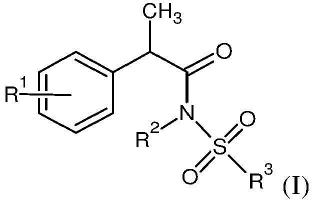 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

According to the present invention, "C₁-C₆-alkyl" represents a linear or branched alkyl chain containing 1 to 6 carbon atoms.

R¹ is preferably selected from benzoyl, isobutyl, and trifluoromethanesulfonyloxy. R¹ is preferably linked to the phenyl ring in 3- or 4-position. According to the most preferred embodiment, R¹ is 3-benzoyl, 4-isobutyl or 4-trifluoromethanesulfonyloxy.

R² is preferably selected from hydrogen or methyl.

R³ is preferably selected from linear or branched C₁-C₆ alkyl, more preferably from linear of branched C₁-C₃ alkyl. According to the most preferred embodiment, R³ is methyl.

The chiral carbon of the compounds of formula (I) is in the RS or R configuration, more preferably it is in the R configuration.

Particularly preferred compounds of formula (I) according to the invention are selected from:
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-[(4-trifluoromethanesulfonyloxy)phenyl]-N-methanesulfonyl propionamide, preferably R(-)-2-[(4-trifluoromethanesulfonyloxy)phenyl]-N-methanesulfonyl propionamide and pharmaceutically acceptable salts thereof, in particular the sodium salt thereof (also known as ladarixin or DF2156A). Compounds of formula (I) are described in WO2000/024710A1 and WO2005/090295A2, which also disclose their method of synthesis. According to a preferred embodiment, said CXCL8 inhibitor has general formula (II): 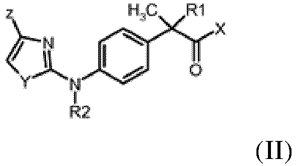 wherein:
   R1 is hydrogen or CH₃;
   X is OH;
   R2 is hydrogen or linear C₁-C₄ alkyl,
   Y is a heteroatom selected from S, O and N,
   Z is selected from linear or branched C₁-C₄ alkyl, linear or branched C₁-C₄ alkoxy, halo C₁-C₃ alkyl and halo C₁-C₃ alkoxy,
   or pharmaceutically acceptable salts thereof.

Preferably, the chiral carbon of the compounds of formula (II) is in the R or S configuration, more preferably it is in the S configuration.

A particularly preferred compound of formula (II) according to the invention is 2-(4-{[4-(trifluoromethyl)-1,3-thiazol-2-yl]amino}phenyl)propanoic acid, preferably (2S)-2-(4-{ [4-(trifluoromethyl)-1,3-thiazol-2-yl] amino} phenyl) propanoic acid or the sodium salt thereof.

Another particularly preferred compound of formula (II) according to the invention is 2-methyl-2-(4-{[4-(trifluoromethyl)-1,3-thiazol-2-yl]amino} phenyl)propanoic acid (DF2726Y) or a pharmaceutically acceptable salt thereof, preferably the sodium salt (DF2726A).

Compounds of formula (II) are described in WO2010/031835A2, which also discloses their method of synthesis.

Preferred CXCL8 inhibitors according to the invention are DF2156A or reparixin, more preferably the CXCL8 inhibitor is reparixin.

According to a preferred embodiment, the present invention is directed to a CXCL8 inhibitor for use in the prevention and/or treatment of ocular mucous membrane pemphigoid in a subject, wherein the CXCL8 inhibitor is 2-(4-isobutylphenyl)propionyl methansulfonamide, preferably R-(-)-2-(4-isobutylphenyl)propionyl methansulfonamide (also known as reparixin) or the lysine salt thereof, wherein the CXCL8 inhibitor is administered systemically to the subject. Preferably, said CXCL8 inhibitor is administered orally to the subject.

According to a further preferred embodiment, the present invention is directed to a CXCL8 inhibitor for use in the prevention and/or treatment of oral mucous membrane pemphigoid in a subject, wherein the CXCL8 inhibitor is selected from 2-(4-isobutylphenyl)propionyl methansulfonamide, preferably R-(-)-2-(4-isobutylphenyl)propionyl methansulfonamide (also known as reparixin) or the lysine salt thereof, and 2-[(4-trifluoromethanesulfonyloxy)phenyl]-N-methanesulfonyl propionamide, preferably R(-)-2-[(4-trifluoromethanesulfonyloxy)phenyl]-N-methanesulfonyl propionamide or a pharmaceutically acceptable salt thereof, in particular the sodium salt thereof (also known as ladarixin or DF2156A), wherein the CXCL8 inhibitor is administered systemically to the subject. Preferably, said CXCL8 inhibitor is administered orally to the subject.

Preferably, the CXCL8 inhibitor for use according to the present invention is administered orally to the subject.

The CXCL8 inhibitor compounds of the present invention may form stable pharmaceutically acceptable acid or base salts with a pharmaceutically acceptable organic or inorganic acid or base, and in such cases administration of a compound as a salt may be appropriate.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Non-toxic physiologically-acceptable salts are preferred, although other salts may be useful, such as in isolating or purifying the product.

The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

The present invention also includes the prodrugs, stereoisomers, isotopelabelled, for example deuterated, derivatives and enantiomers of the CXCL8 inhibitor compounds described above.

As used herein the term "prodrug" refers to an agent, which is converted into the parent drug in vivo by some physiological chemical process (e.g., a prodrug on being brought to the physiological pH is converted to the desired drug form). Prodrugs are often useful because, in some situations, they may be easier to administer than the parent drug. For instance, they may be bioavailable by oral administration whereas the parent drug is not. The prodrug may also have improved solubility in pharmacological compositions over the parent drug. An example, without limitation, of a prodrug would be a compound of the present invention that is administered as an ester (the "prodrug") to facilitate transmittal across a cell membrane where water solubility is not beneficial, but then that it is metabolically hydrolysed once inside the cell where water solubility is beneficial.

Prodrugs have many useful properties. For example, a prodrug may be more water-soluble than the ultimate drug, thereby facilitating intravenous administration of the drug. A prodrug may also have a higher level of oral bioavailability than the ultimate drug. After administration, the prodrug is enzymatically or chemically cleaved to deliver the ultimate drug in the blood or tissue.

Ester prodrugs of the CXCL8 inhibitor compounds disclosed herein are specifically contemplated. While not intending to be limiting, an ester may be an alkyl ester, an aryl ester, or a heteroaryl ester. The term alkyl has the meaning generally understood by those skilled in the art and refers to linear, branched, or cyclic alkyl moieties. C1-6 alkyl esters are particularly useful, where alkyl part of the ester has from 1 to 6 carbon atoms and includes, but is not limited to, methyl, ethyl, propyl, isopropyl, n-butyl, sec-butyl, iso-butyl, t-butyl, pentyl isomers, hexyl isomers, cyclopropyl, cyclobutyl, cyclopentyl, cyclohexyl, and combinations thereof having from 1 to 6 carbon atoms.

Certain CXCL8 inhibitor compounds may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers.

Particular optical isomers can be obtained by resolution of the racemic mixtures according to conventional processes, e.g., by formation of diastereomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

As will be discussed in the experimental section, the present inventors have shown that systemic oral administration of a CXCL8 inhibitor in an animal model of MMP results in amelioration of both ocular and oral MMP.

The invention is also directed to a pharmaceutical composition comprising a CXCL8 inhibitor, as described above, and at least one pharmaceutically acceptable excipient or carrier for use in the prevention and/or treatment of ocular mucous membrane pemphigoid and/or oral mucous membrane pemphigoid in a subject, wherein said pharmaceutical composition is administered systemically to the subject.

Preferably, said pharmaceutical composition is administered orally to the subject.

According to a preferred embodiment, said pharmaceutical composition is for use in the prevention and/or treatment of ocular mucous membrane pemphigoid in a subject, wherein said pharmaceutical composition is administered systemically to the subject. Preferably, said pharmaceutical composition is administered orally to the subject.

According to a preferred embodiment, said pharmaceutical composition is for use in the prevention and/or treatment of oral mucous membrane pemphigoid in a subject, wherein said pharmaceutical composition is administered systemically to the subject. Preferably, said pharmaceutical composition is administered orally to the subject.

According to a preferred embodiment, said pharmaceutical composition is for use in the prevention and/or treatment of ocular mucous membrane pemphigoid and oral mucous membrane pemphigoid in a subject, wherein said pharmaceutical composition is administered systemically to the subject. Preferably, said pharmaceutical composition is administered orally to the subject.

According to a preferred embodiment, said pharmaceutical composition is selected from a liquid composition, such as a solution or a suspension, and a solid composition, such as a capsule or a tablet.

The administration of the pharmaceutical composition of the present invention to a patient may comprise from one to several oral administrations per day (for example, two times a day (BID) or four times a day (QID)).

Preferably, the pharmaceutical composition is a composition suitable for oral administration to the subject.

Accordingly, the invention is further directed to an oral composition comprising a therapeutically effective amount of a CXCL8 inhibitor, as described above, and at least one pharmaceutically acceptable excipient or carrier, for use in the prevention and/or treatment of ocular mucous membrane pemphigoid and/or oral mucous membrane pemphigoid in a subject.

According to a preferred embodiment, said oral composition is for use in the prevention and/or treatment of ocular mucous membrane pemphigoid in a subject.

According to a preferred embodiment, said oral composition is for use in the prevention and/or treatment of oral mucous membrane pemphigoid in a subject. According to a preferred embodiment, said oral composition is for use in the prevention and/or treatment of ocular mucous membrane pemphigoid and oral mucous membrane pemphigoid in a subject.

Preferably, said oral composition is selected from a liquid composition, such as a suspension or a solution, and a solid composition, such as a capsule or a tablet. A "therapeutically effective amount" according to the present invention means an amount sufficient to achieve treatment or prevention of the disease. Determination of the effective amounts is well within the capability of those skilled in the art based upon the achievement of a desired effect. An effective amount will depend on factors including, but not limited to, the weight of a subject and/or the degree of the disease or unwanted condition from which a subject suffers.

As used herein, the term "pharmaceutically acceptable excipient or carrier" includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such as propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, salts for regulating osmotic pressure, emulsifiers, sweeteners, colorants, flavourings and the like.

The amount of a CXCL8 inhibitor or the pharmaceutically acceptable salt thereof in the pharmaceutical composition of the present invention can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

The invention relates also to the use of a CXCL8 inhibitor, as above defined, in the manufacture of a medicament for the prevention and/or treatment of ocular mucous membrane pemphigoid and/or oral mucous membrane pemphigoid, wherein said CXCL8 inhibitor is administered systemically. According to a preferred embodiment, said CXCL8 inhibitor is administered orally.

The invention is also directed to a method of preventing and/or treating ocular mucous membrane pemphigoid and/or oral mucous membrane pemphigoid, which comprises administering an effective amount of one or more CXCL8 inhibitor, as above defined, to a subject in need thereof, wherein said one or more CXCL8 inhibitor is administered systemically to the subject.

According to a preferred embodiment, said one or more CXCL8 inhibitor is administered orally to the subject.

The invention is further illustrated by the following example.

### EXAMPLE

A study was designed to investigate and show the effect of pharmacological inhibition of CXCR1/2 exerted by the systemic administration of the compounds DF2156A and reparixin in a mouse model of MMP.

### Materials and methods

### Test compounds

| **Compound** | **Lot.** | **Expiry date** | **Supplier** | **Storage** |
|---|---|---|---|---|
| Ladarixin Sodium Salt (DF2156A) | 117116001 | Not applicable | Dompé S.p.A. | +4°C |
| Reparixin Lysine Salt | MA905-30 | Not applicable | Dompé S.p.A. | +4°C |
| Vehicle | Not applicable | Not applicable | Dompé S.p.A. | +4°C |
| Methylprednisolone | sourced by UKSH, Lübeck | July 2024 | Sanofi | 20-25°C |

| | | | | |
|---|---|---|---|---|
| Animal model - Mice | | | | |

Adult C57B1/6 (B6) mice (male and female) aged at least 6 weeks old were used. Animals were maintained on a 12-h light-dark cycle at the animal facility of the University of Lübeck. Mice were held under SPF conditions and fed acidified drinking water and standard chow ad libitum. Protocols were approved by the Animal Rights Commission of the Ministry of Agriculture and Environment, Schleswig-Holstein.

### Generation, isolation and characterization of anti-mLAMα3 IgG

For production of anti-mLAMα,3 IgG, New Zealand White rabbits were immunized subcutaneously with 250µg of an equimolar mixture of the 2 purified recombinant proteins (aa1656-1985 and aa2756-3330 of murine Laminin alpha3 chain, produced in E. coli as disclosed by Heppe, E.N., et al., J Invest Dermatol, 2017, 137, 1709-1718) suspended in complete Freund's adjuvant. The animals were boosted twice with the same protein preparation in incomplete Freund's adjuvant. Immune sera were obtained at regular intervals and characterized by immunofluorescence (IF) microscopy on cryosections of murine skin. IgG from rabbits immunized with recombinant fragments of murine mLAMα3 and from non-immunized rabbits was purified by affinity chromatography using protein G sepharose affinity chromatography (Amersham Biosciences, Heidelberg, Germany). Reactivity of IgG fractions was analyzed by IF microscopy on murine skin (Sitaru et al., J Immunol 2006, 177: 3461-8). In addition, each batch of anti-mLAMα,3 IgG was characterized *in vivo* regarding its capability to induce experimental MMP in C57B1/6 (B6) mice (WP1.1). From this experiment, the amount of antibodies that leads to moderate experimental MMP was determined; i.e. the amount needed to induce disease development with moderate involvement of the conjunctiva (i.e. score of 1-3 in a maximum of 50% of the animals on day 12) in the model of antibody transfer-induced MMP. For this purpose, the intensity of the separation of the conjunctiva is determined histologically. The body surface area affected should be between 3-8% at the concentration used. The conjunctiva score was set histologically post-mortem and was in a range of 0-4, depending on the intensity of the separation of the conjunctiva. The amount needed was determined to be 5 mg/injection of anti-mLAMα,3 IgG.

### Study design

### Induction of experimental MMP by repetitive injections of anti-mLAMα3 IgG and treatment protocol

To test if DF2156A at 15 mg/kg body weight and Reparixin at 30 mg/kg body weight influence the severity of oral and conjunctival involvement in experimental MMP, the disease was induced by repetitive s.c. injections of anti-mLAMα3 IgG (5mg/mouse) on alternating days (0, 2, (...) and 10) into adult B6 mice. Mice were treated throughout the entire experiment once daily for DF2156A and twice daily for Reparixin via feeding needles (p.o.). Mice treated with solvent (vehicle) twice daily (p.o.) or once daily with methylprednisolone (MP) via i.p. injections were used as treatment controls. Mice s.c. injected with IgG isolated from normal rabbit serum (NR IgG) served as negative control.

### Evaluation of conjunctival lesions

The primary endpoint of this experiment was the extent of conjunctival lesions as determined by lesional histopathology (H&E stain) on day 12 and 28 following an established scoring system (Heppe, E.N., et al., J Invest Dermatol, 2017, 137, 1709-1718). In detail, biopsies from the palpebral conjunctiva were taken on day 12 and day 28 and paraffine embedded. 4.5 µm thick sections from 3 different depths of the biopsies were cut in triplicate and H&E stained for further quantification. Among the H&E stained histologies those with a palpebral conjunctival-epithelium with more than 1000 µm were used for quantification. For this score the length of the split formation (split = epithelia separation from the underlying dermal structures) is measured. The split length is categorized to a score of 0-4 with no split = 0, less than 100 µm = 1, less than 200 µm = 2, less than 300 µm = 3, more or equal 300 µm = 4. Splits that occurred at the end of the tissue were excluded, because they were most likely to be of artificial nature due to the cutting. The longest split out of 9 possible sections is decisive for the final score.

The endpoint was analyzed on day 12 in the following groups:
▪ normal rabbit IgG (n=10)
▪ Anti-mLAMα,3 IgG + solvent (positive control) (n=14)
▪ Anti-mLAMα,3 IgG + MP (reference treatment) (n=13)
▪ Anti-mLAMα,3 IgG + DF2156A (n=13)
▪ Anti-mLAMα,3 IgG + reparixin (n=13)

The endpoint was analyzed on day 28 in the following groups:
▪ normal rabbit IgG (n=9)
▪ Anti-mLAMα,3 IgG + solvent (positive control) (n=12)
▪ Anti-mLAMα,3 IgG + MP (reference treatment) (n=11)
▪ Anti-mLAMα,3 IgG + DF2156A (n=13)
▪ Anti-mLAMα,3 IgG + reparixin (n=12)

The experiments were performed at two independent time points for each day 12 and day 28, including 7 mice/group and 5 mice/normal rabbit IgG.

### Extent of inflammatory infiltration of conjunctival samples (H&E histology)

The extent of inflammatory infiltration of palpebral conjunctival samples was quantified on H&E stained sections. 2 visual fields were examined by a blinded scorer for inflammatory infiltrate with regard to the severity. The mean for each sample was used. The maximum score is 4.

The endpoint was analyzed on day 12 in the following groups:
▪ normal rabbit IgG (n=9)
▪ Anti-mLAMα,3 IgG + solvent (positive control) (n=11)
▪ Anti-mLAMα,3 IgG + MP (reference treatment) (n=11)
▪ Anti-mLAMα,3 IgG + DF2156A (n=11)
▪ Anti-mLAMα,3 IgG + reparixin (n=12)

The endpoint was analyzed on day 28 in the following groups:
▪ normal rabbit IgG (n=9)
▪ Anti-mLAMα,3 IgG + solvent (positive control) (n=12)
▪ Anti-mLAMα,3 IgG + MP (reference treatment) (n=11)
▪ Anti-mLAMα,3 IgG + DF2156A (n=12)
▪ Anti-mLAMα,3 IgG + reparixin (n=12)

### Severity of oral lesions

The extent of oral lesions was determined by endoscopy (Videomed, München, Germany) on days 12 and 28 following an established scoring system (Heppe, E.N., et al., J Invest Dermatol, 2017, 137, 1709-1718). In detail, each affected mouth-quarter of the mouse is counted as one point if there are lesions/blisters/crusts/erosions. The quarters are defined as: left buccal mucosa, right buccal mucosa, hypopharynx, tongue. The maximum score is 4.

### Weight loss

The weight of the mice was measured on days 4, 8, 12, 16, 20, 24, and 28. The body weight loss is a marker for disease severity and/or reduced tolerance to the treatment.

### Statistical analysis

For statistical analysis GraphPad Prism (version 8.4.3) was used. For comparison of treatment effects in multiple groups ANOVA was used. To isolate the group or groups that differ from others, Dunnett's or Holm Sidaks multiple comparison were used when appropriate. In special cases a mixed-effects analysis (missing data) or an uncorrected Fishers LSD method was performed.

### Results

### Systemic application of reparixin reduces split formation in the palpebral conjunctiva in experimental MMP

Injection of rabbit anti-mLAMα,3 IgG into adult B6 mice persistently lead to induction of experimental MMP within 4-8 days after the first IgG injection.

After 12 days of systemic application of the drugs via oral gavage a significant reduction of the conjunctival split formation for the treatment with reparixin compared to mice that received the vehicle was determined (figure 1A). Also, a significantly decreased inflammatory infiltrate was detected for this group (figure 1B). In mice that received MP (i.p, once daily) as reference treatment and reparixin the extent of affected eye area of experimental MMP was reduced at day 12. In mice injected with NR-IgG no conjunctival lesions were observed compared to the other groups on day 12 (figure 2).

Treatment over 28 days with reparixin confirmed the results obtained at day 12. Indeed, a statistically significant reduction of conjunctival split formation and a reduced inflammatory infiltrate was found in the mice treated with reparixin when compared to vehicle treated mice (figures 3A, 3B). Mice that just received NR-IgG from healthy rabbits did not show conjunctival lesions compared to the other groups on day 28 (figure 4).

Systemic treatment with reparixin showed overall a benefit for diseased mice with regard to the conjunctival split formation and the inflammation of this tissue already at day 12 and until day 28.

### Systemic application of reparixin and DF2156A reduces oral lesions in experimental MMP

Reparixin and DF2156A treatment reduced oral lesions in experimental MMP after 12 days (figure 6) and showed a trend for reduction at 28 days when compared to vehicle treated mice (figure 8).

### Systemic application of reparixin and DF2156A is better tolerated than reference treatment with MP in terms of weight loss

At day 12, no statistically significant differences were observed between body weight in treatments group compared to mice treated with vehicle (figure 5). However, a trend showing a reduced weight loss was observed for groups treated with DF2156A and reparixin compared to the group treated with MP, suggesting that the treatment with DF2156A and reparixin is better tolerated than the reference treatment with MP. The same trend was observed also at day 28 (figure 7), thus confirming that the treatment with reparixin and DF2156A is overall better tolerated than the reference treatment with MP.

### Conclusions

The data obtained show that systemic treatment with reparixin reduces conjunctival split formation and oral lesions already at day 12. Both scores were healed by approximately one third by reparixin treatment.

Reparixin also reduced the inflammation of the palpebral conjunctiva at day 12. A longer lasting treatment until day 28 consistently confirmed the positive impact on the conjunctival split formation and on the palpebral conjunctiva. The systemic administration of reparixin and DF2156A reduced the oral lesions in experimental MMP significantly after 12 days of treatment.

DF2156A and reparixin were found to be better tolerated than the reference treatment with MP, as shown by the reduced weight loss in animals treated with these compounds when compared with animals treated with the reference treatment MP.

## Claims

1. A CXCL8 inhibitor for use in the prevention and/or treatment of ocular mucous membrane pemphigoid and/or oral mucous membrane pemphigoid in a subject, wherein said CXCL8 inhibitor is administered systemically to the subject.

2. A CXCL8 inhibitor for use as claimed in claim 1, wherein said CXCL8 inhibitor is administered orally to the subject.

3. A CXCL8 inhibitor for use according to claim 1 or claim 2, which is a CXCL8 receptor(s) inhibitor selected from a CXCR1- or a CXCR1/2-inhibitor.

4. A CXCL8 inhibitor for use according to any one of claims 1 to 3, which is an antagonist of the CXCR1 receptor or an antagonist of both CXCR1 and CXCR2 receptors.

5. A CXCL8 inhibitor for use according to any one of claims 1 to 4, which is a CXCL8 receptor(s) inhibitor selected from an allosteric inhibitor or an orthosteric antagonist of CXCR1 receptor or of both CXCR1 and CXCR2 receptors.

6. A CXCL8 inhibitor for use according to any one of claims 1 to 5, having general formula (I): 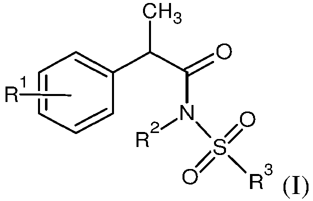 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

7. A CXCL8 inhibitor for use according to claim 6, wherein the chiral carbon of the compounds of formula (I) is in the R configuration.

8. A CXCL8 inhibitor for use according to any one of claims 1 to 5, having general formula (II): 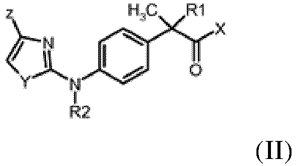 wherein:
R1 is hydrogen or CH₃;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or pharmaceutically acceptable salts thereof.

9. A CXCL8 inhibitor for use according to claim 8, wherein the chiral carbon of the compounds of formula (II) is in the S configuration.

10. A CXCL8 inhibitor for use according to any one of claims 1 to 9, wherein said CXCL8 inhibitor is selected from the group consisting of:
- the anti-CXCL-8 antibodies ABCream, BMS-986253 and ABX-IL-8;
- RP-72, PAC-G-31-P, SCH-N,
- Navarixin, having formula: 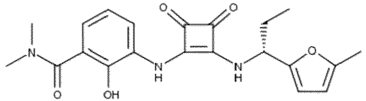
- SX-517, having formula: 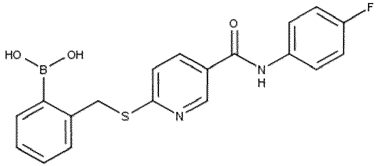
- SX-576, having formula: 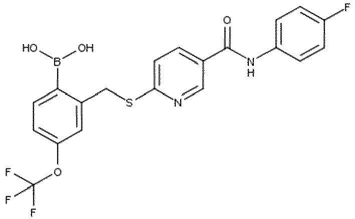
- SX-682, having formula: 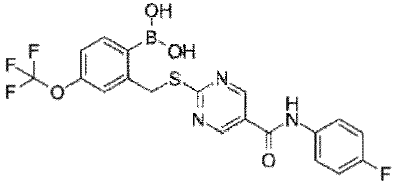
- compounds having formula: 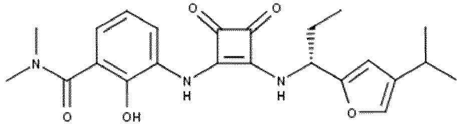 or 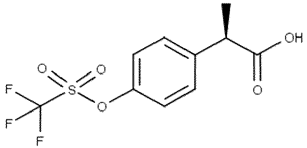 or 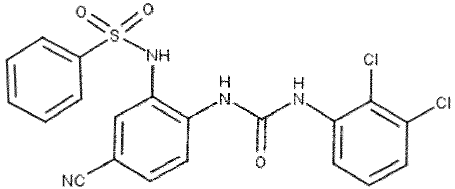 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) as defined in claims 6 to 9.

11. A CXCL8 inhibitor for use according to claim 6 or 7, having general formula (I) which is selected from:
- 2-(4-isobutylphenyl)propionyl methansulfonamide, preferably R-(-)-2-(4-isobutylphenyl)propionyl methansulfonamide and pharmaceutically acceptable salts thereof, preferably the lysine salt thereof, and
- 2-[(4-trifluoromethanesulfonyloxy)phenyl]-N-methanesulfonyl propionamide, preferably R(-)-2-[(4-trifluoromethanesulfonyloxy)phenyl]-N-methanesulfonyl propionamide and pharmaceutically acceptable salts thereof, in particular the sodium salt thereof.

12. A CXCL8 inhibitor for use according to claim 11, which is the lysine salt of 2-(4-isobutylphenyl)propionyl methansulfonamide, preferably R-(-)-2-(4-isobutylphenyl)propionyl methansulfonamide.

13. A CXCL8 inhibitor for use according to claim 8 or 9, having general formula (II) which is 2-(4-{ [4-(trifluoromethyl)-1,3-thiazol-2-yl]amino}phenyl)propanoic acid, preferably (2S)-2-(4-{ [4-(trifluoromethyl)-1,3-thiazol-2-yl] amino} phenyl) propanoic acid or the sodium salt thereof.

14. A pharmaceutical composition comprising a CXCL8 inhibitor as defined 7for use in the prevention and/or treatment of ocular mucous membrane pemphigoid and/or oral mucous membrane pemphigoid in a subject, wherein said pharmaceutical composition is administered systemically to the subject.

15. The pharmaceutical composition for use according to claim 14, wherein said pharmaceutical composition is administered orally to the subject.
